# FASCICULE DE BREVET EUROPEEN

(11) **EP 2 343 040 B1**
(45) Date de publication et mention de la délivrance du brevet: **23.11.2016**
(21) Numéro de dépôt: 10195743.9
(22) Date de dépôt: 17.12.2010
(51) Int. Cl.: A61K 8/58, A61K 8/46, A61K 8/39, A61K 8/898, A61Q 5/02, A61Q 5/12

(54) **Composition cosmétique comprenant au moins un compose organique du silicium, au moins un tensioactif anionique et au moins une silicone aminée ainsi qu'un procède mettant en oeuvre ladite composition**
Kosmetische Zusammensetzung, die mindestens eine organische Komponente aus Silizium, mindestens ein anionisches Tensid und mindestens ein Aminsilikon enthält, sowie Verfahren zum Einsatz dieser Zusammensetzung
Cosmetic composition containing at least one organic silicon compound, at least one anionic surface-active agent and at least one amine silicone as well as a method implementing said composition

(30) Priorité: 23.12.2009 FR 0959488
(43) Date de publication de la demande: 13.07.2011
(73) Titulaire: L'Oréal, 75008 Paris (FR)
(72) Inventeur: Viravau, Valérie, 75001, Paris (FR); Aires, Carine, 75014, Paris (FR); Drillon, Damien, 75008, Paris (FR); Mellul, Myriam, 75014, Paris (FR)
(74) Mandataire: Dossmann, Gérard

(56) Documents cités:
- EP-A2- 0 413 416
- WO-A2-2009/043841
- FR-A1- 2 789 896
- FR-A1- 2 864 779
- FR-A1- 2 930 439

## Description

La présente invention concerne une composition de nettoyage et de conditionnement des fibres kératiniques, en particulier des fibres kératiniques humaines telles que les cheveux, comprenant, dans un milieu cosmétiquement acceptable, un ou plusieurs composés organiques du silicium, un ou plusieurs tensioactifs anioniques et une ou plusieurs silicones aminées particulières. La présente invention concerne également un procédé de traitement cosmétique des fibres kératiniques ainsi qu'une utilisation mettant en oeuvre ladite composition cosmétique.

Pour le nettoyage et/ou le lavage des matières kératiniques telles que les cheveux, l'utilisation de compositions détergentes (telles que les shampooings) à base essentiellement d'agents tensioactifs classiques de type notamment anionique, non ionique et/ou amphotère, mais plus particulièrement de type anionique, est courante. Ces compositions sont appliquées sur cheveux mouillés et la mousse générée par massage ou friction avec les mains permet, après rinçage à l'eau, l'élimination des diverses salissures initialement présentes sur les cheveux ou la peau.

Ces compositions de base présentent certes un bon pouvoir lavant mais les propriétés cosmétiques intrinsèques qui leur sont attachées restent toutefois assez faibles, notamment en raison du fait que le caractère relativement agressif d'un tel traitement de nettoyage peut entraîner à la longue sur la fibre capillaire des dommages plus ou moins marqués liés en particulier à l'élimination progressive des lipides ou protéines contenues dans ou à la surface de cette dernière.

Aussi, pour améliorer les propriétés cosmétiques des compositions détergentes ci-dessus, et plus particulièrement de celles qui sont appelées à être appliquées sur des cheveux sensibilisés (c'est-à-dire les cheveux qui se trouvent généralement abîmés ou fragilisés par l'action des agents atmosphériques extérieurs tels que la lumière et les intempéries, et/ou des traitements mécaniques ou chimiques tels que le brossage, le peignage, les teintures, les décolorations, les permanentes et/ou les défrisages), il est maintenant usuel d'introduire dans ces dernières des agents cosmétiques complémentaires dits agents conditionneurs destinés principalement à réparer ou limiter les effets néfastes ou indésirables induits par les différents traitements ou agressions que subissent, de manière plus ou moins répétés, les fibres capillaires. Ces agents de conditionnement peuvent bien entendu également améliorer le comportement cosmétique des cheveux naturels.

Dans ce but, on a déjà proposé d'utiliser des composés organiques cosmétiquement actifs tels que des polymères cationiques et des silicones en tant qu'agents de conditionnement, dans des compositions cosmétiques détergentes tels que des shampooings, pour conférer aux cheveux des propriétés cosmétiques satisfaisantes, en particulier de la brillance, de la douceur, de la souplesse, de la légèreté, un toucher naturel ainsi qu'une aptitude au démêlage améliorée.

Cependant, l'emploi de ces composés dans des compositions cosmétiques de lavage et de conditionnement ne procure pas aux cheveux des propriétés coiffantes satisfaisantes et durables. En effet, ces compositions procurent généralement des effets coiffants, tels que des effets de maintien, de corps et/ou de discipline des cheveux, qui restent insuffisants et qui ont tendance à s'estomper après un lavage des cheveux avec un shampooing classique.

Or on a constaté que les consommateurs sont de plus en plus à la recherche de compositions de lavage qui soient non seulement capables de conditionner les cheveux de manière convenable également capables de procurer des effets coiffants satisfaisants et durables.

Ainsi des compositions destinées au lavage et au conditionnement des cheveux qui comprennent des composés organiques du silicium, tel que le 3-aminopropyltriéthoxysilane, ont été développées afin de pouvoir répondre à ces exigences. Ces compositions de lavage permettent de conditionner les cheveux, notamment en leur apportant un toucher doux satisfaisant, tout en conférant des effets coiffants marqués et durables.

De plus, ces compositions se sont révélées particulièrement avantageuses car elles permettent de faciliter la mise en forme des cheveux fins et de conférer des effets coiffants intéressants aux cheveux bouclés ou frisés, notamment en améliorant le dessin et le contrôle des boucles.

Toutefois, les compositions de lavage comprenant de tels composés organiques du silicium présentent généralement l'inconvénient d'évoluer sensiblement au cours du temps dans des conditions normales de stockage en fonction de la température, notamment au niveau de leur viscosité et de leur aspect visuel. En d'autres termes, ces compositions ne s'avèrent pas stables ce qui se traduit le plus souvent par un aspect visuel trouble ainsi que par une texture non satisfaisante au stockage.

En effet, il a été constaté que les composés organiques du silicium, tel que le 3-aminopropyltriéthoxysilane, n'étaient pas compatibles chimiquement avec la totalité des tensioactifs, notamment des tensioactifs anioniques, qui peuvent être présents dans les compositions de lavage, ce qui engendre les problèmes de stabilité rencontrés.

Par ailleurs, on a observé que l'introduction de certains composés organiques du silicium, en particulier les dérivés aminés tels que le 3-aminopropyltriéthoxysilane, dans des compositions de lavage, qui présentent généralement un pH allant de 4 à 7, engendre également des problèmes de stabilité du fait du caractère alcalin de ces composés.

Il existe donc un réel besoin de mettre au point des compositions cosmétiques destinées au nettoyage et au conditionnement des fibres kératiniques contenant des composés organiques du silicium, ces compositions ne présentant pas l'ensemble des inconvénients décrits ci-dessus, c'est-à-dire qui sont stables dans le temps et qui permettent de conditionner les cheveux de manière satisfaisante tout en apportant des effets coiffants puissants durables, notamment en termes de masse, de corps, de texturisation des cheveux.

La demanderesse a découvert, de façon surprenante, qu'il était possible de formuler des compositions détergentes et conditionnantes, ayant les propriétés recherchées, comprenant un ou plusieurs composés organiques du silicium tels que définis ci-après, un ou plusieurs tensioactifs anioniques et une ou plusieurs silicones aminées non quaternisées.

En effet, il a été constaté que l'utilisation de silicones aminées ne comportant pas dans leur structure un groupement ammonium quaternaire dans des compositions cosmétiques comprenant un ou plusieurs composés organiques du silicium et un ou plusieurs tensioactifs anioniques permet de les rendre stables au stockage aussi bien à température ambiante (20-25°C) qu'à 45°C, notamment au niveau de leur aspect visuel et de leur viscosité.

Par « stables » au sens de la présente invention, on entend que l'aspect visuel ainsi que la viscosité de ces compositions n'évoluent pas sensiblement au cours du temps dans des conditions standards de tests de stockage par exemple pendant les 2 mois à température ambiante (20°C-25°C), et/ou à 45°C et/ou à 4°C, qui suivent leur fabrication.

De plus, les compositions selon l'invention conduisent à un traitement satisfaisant des cheveux leur conférant ainsi un toucher doux satisfaisant, une aptitude au démêlage améliorée, de la douceur et la souplesse.

Par ailleurs, les compositions conformes à la présente invention apportent aux cheveux des effets coiffants puissants, en leur apportant notamment de la masse, du corps et/ou de la discipline et ceci de manière durable.

De plus, les compositions selon l'invention permettent de faciliter la mise en forme des cheveux, en particulier des cheveux fins, et de conférer des effets coiffants améliorés aux cheveux bouclés, notamment en terme de dessin et de contrôle des boucles, et ceci de manière durable.

La présente invention concerne notamment une composition cosmétique pour le lavage et le conditionnement des fibres kératiniques, en particulier des fibres kératiniques humaines telles que les cheveux, comprenant dans un milieu cosmétiquement acceptable :
i) un ou plusieurs composés organiques du silicium choisis parmi les silanes comprenant un atome de silicium et les siloxanes comprenant deux ou trois atomes de silicium, lesdits composés organiques du silicium comportant en outre une ou plusieurs fonctions chimiques basiques et un ou plusieurs groupes hydroxyles ou hydrolysables par molécule ;
ii) un ou plusieurs tensioactifs anioniques ; et
iii) une ou plusieurs silicones aminées non quaternisées différentes des composés i).

La présente invention concerne également un procédé de traitement cosmétique des cheveux, en particulier de lavage et de conditionnement, comprenant l'application sur lesdites fibres de la composition selon l'invention.

Elle concerne aussi l'utilisation de la composition selon l'invention comme shampoing pour le lavage et le conditionnement des cheveux.

D'autres objets et caractéristiques, aspects et avantages de l'invention apparaîtront encore plus clairement à la lecture de la description et des exemples qui suivent.

Les composés organiques du silicium utilisés dans la composition selon l'invention sont choisis parmi les organosilanes comprenant un atome de silicium et les organosiloxanes comportant deux ou trois atomes de silicium, de préférence deux atomes de silicium. Ils doivent en outre comporter une ou plusieurs fonctions chimiques basiques, et de préférence une seule fonction chimique basique. La fonction chimique basique peut correspondre à toute fonction conférant un caractère basique au composé de silicium et est de préférence une fonction amine telle qu'une fonction amine primaire, secondaire ou tertiaire. Les composés du silicium selon l'invention, peuvent comporter éventuellement d'autres fonctions, telles que, par exemple, une fonction acide ou une fonction halogène.

Le ou les composés organiques du silicium utilisés dans la composition selon invention, comportent en outre deux ou plusieurs groupes hydrolysables ou hydroxyles par molécule. Les groupes hydrolysables sont de préférence des groupes alcoxy, aryloxy ou halogène. Ils peuvent également, éventuellement, comporter d'autres fonctions chimiques telles que des fonctions acides.

Selon un mode de réalisation particulier, le ou les organosilanes utilisées dans la composition selon l'invention sont choisis parmi les composés de formule (I) : 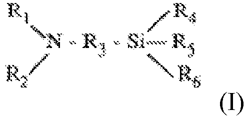 dans laquelle :
R₄ représente un halogène, un groupe OR' ou R'₁ ;
R₅ représente un halogène, un groupe OR" ou R'₂ ;
R₆ représente un halogène, un groupe OR'" ou R'₃ ;
R₁, R₂, R₃, R', R", R"', R'₁, R'₂, R'₃ représentent, indépendamment les uns des autres, un groupe hydrocarboné saturé ou insaturé, linéaire ou ramifié, portant éventuellement des groupes chimiques supplémentaires, R₁, R₂, R', R" et R'" pouvant en outre désigner l'hydrogène, et deux au moins des groupes R₄, R₅ et R₆ désignant respectivement OR', OR" et OR"', deux au moins des groupes R', R" et R"' étant différents de l'hydrogène.

De préférence, les groupes R₁, R₂, R', R'₁, R'₂, R'₃, R"et R'" sont choisis parmi les radicaux alkyle de C₁-C₁₂, aryle de C₆ à C₁₄, alkyle de C₁ à C₈-aryle de C₆ à C₁₄, et aryle de C₆ à C₁₄-alkyle de C₁ à C₈.

Selon un autre mode de réalisation particulier, le ou les organosiloxanes utilisées dans la composition selon l'invention sont choisis parmi les composés de formule (II) : 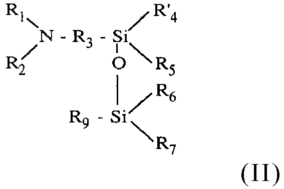 dans laquelle :
R₁, R₂, R₃, R₅ et R₆ sont définis comme précédemment ;
R'₄ représente un atome d'halogène ou un groupe OR₁₁ ;
R₇ représente un atome d'halogène, un groupe OR₁₀ ou R"₁ ;
R₉ représente un atome d'halogène, un groupe OR₈, R"₂ ou R₃NR₁R₂ ; R"₁, R"₂, R₈, R₁₀ et R₁₁ représente un groupe hydrocarboné, saturé ou insaturé, linéaire ou ramifié, portant éventuellement des groupes chimiques supplémentaires, les groupes R₁₁, R₁₀ et R₈ pouvant en outre représenter un atome d'hydrogène ; l'un au moins des groupes R₆, R₇ et R₉ désignant un atome d'halogène, un groupe OR"', OR₁₀ ou OR₈.

De préférence, les groupes R"₁, R"₂, R₈ ou R₁₀ et R₁₁ sont choisis parmi les radicaux alkyle de C₁-C₁₂, aryle de C₆ à C₁₄, alkyle de C₁ à C₈-aryle de C₆ à C₁₄, et aryle de C₆ à C₁₄-alkyle de C₁ à C₈.

En particulier, l'atome d'halogène est un atome de chlore.

Le ou les composés organiques du silicium utilisés dans la composition selon invention sont de préférence des organosilanes choisis parmi les composés de formule (III) : 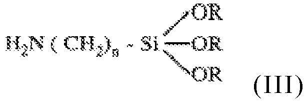 dans laquelle les radicaux R, identiques ou différents, sont choisis parmi les radicaux alkyle en C₁-C₆, de préférence en C₁-C₂, et n est un nombre entier de 1 à 6, de préférence de 2 à 4.

De préférence, les silanes ou les siloxanes sont solubles dans l'eau et encore plus préférentiellement solubles à la concentration de 2%, mieux à la concentration de 5% et encore mieux à la concentration de 10% en poids dans l'eau à la température de 25°C±5°C et à la pression atmosphérique. Par soluble, on entend la formation d'une phase macroscopique unique.

De façon particulièrement préférée, le composé organique du silicium présent dans la composition selon l'invention est le 3-aminopropyl triéthoxysilane.

Le ou les composés organiques du silicium peuvent être présents dans la composition selon l'invention dans une teneur allant de 0,01 à 10 % en poids, de préférence dans une teneur en poids allant de 0,1 à 5% en poids, et plus préférentiellement dans une teneur en poids allant de 0,2 à 2% en poids, par rapport au poids total de la composition.

Comme indiqué précédemment, la composition cosmétique selon la présente invention contient en outre un ou plusieurs tensioactifs anioniques.

Les tensioactifs anioniques utilisés dans les compositions de l'invention sont notamment choisis parmi les sels, en particulier les sels de métaux alcalins tels que les sels de sodium, les sels d'ammonium, les sels d'amines, les sels d'aminoalcools ou les sels de métaux alcalino-terreux, par exemple, de magnésium, des types suivants : les alkylsulfates, les alkyléthersulfates, les alkylamidoéthersulfates, les alkylarylpolyéthersulfates, les monoglycéride-sulfates, les alkylsulfonates, les alkylamidesulfonates, les alkylarylsulfonates, les α-oléfine-sulfonates, les paraffine-sulfonates, les alkylsulfosuccinates, les alkyléthersulfosuccinates, les alkylamide-sulfosuccinates, les alkylsulfo-acétates, les acylsarcosinates et les acylglutamates, les groupes alkyle et acyle de tous ces composés comportant de 6 à 24 atomes de carbone et le groupe aryle désignant de préférence un groupe phényle ou benzyle.

On peut également utiliser les monoesters d'alkyle en C₆₋₂₄ et d'acides polyglycoside-dicarboxyliques tels que les glucoside-citrates d'alkyle, les polyglycoside-tartrates d'alkyle et les polyglycoside-sulfosuccinates d'alkyle, les alkylsulfosuccinamates, les acyliséthionates et les N-acyltaurates, le groupe alkyle ou acyle de tous ces composés comportant de 12 à 20 atomes de carbone.

Un autre groupe d'agents tensioactifs anioniques utilisables dans les compositions de la présente invention est celui des acyl-lactylates dont le groupe acyle comporte de 8 à 20 atomes de carbone.

En outre, on peut encore citer les acides alkyl-D-galactoside-uroniques et leurs sels ainsi que les acides (alkyl en C₆₋₂₄)éther-carboxyliques polyoxyalkylénés, les acides (alkyl en C₆₋₂₄)(aryl en C₆₋₂₄)éther-carboxyliques polyoxyalkylénés, les acides (alkyl en C₆₋₂₄)amidoéther-carboxyliques polyoxyalkylénés et leurs sels, en particulier ceux comportant de 2 à 50, mieux de 2 à 10 et encore mieux de 2 à 5 motifs oxyde d'éthylène, et leurs mélanges.

On utilise de préférence les alkylsulfates, les alkyléthersulfates, les alkyléthercarboxylates et leurs mélanges, en particulier sous forme de sels de métaux alcalins ou alcalino-terreux, d'ammonium, d'amine ou d'aminoalcool.

De préférence, le ou les tensioactifs anioniques utilisés dans la composition cosmétique selon l'invention sont choisis parmi les alkyléthersulfates, de préférence en C₁₂-C₁₄, et plus particulièrement comprenant de 2 à 3 moles d'oxyde d'éthylène et les N-acyl taurates.

Les tensioactifs anioniques peuvent être présents dans une teneur allant de 1 à 25% en poids, de préférence dans une teneur allant de 3 à 20% en poids, et encore plus préférentiellement dans une teneur allant de 5 à 15% en poids, par rapport au poids total de la composition cosmétique selon l'invention.

La composition selon la présente invention contient en outre une ou plusieurs silicones aminées non quaternisées différentes des silanes ou siloxanes (i).

Par « silicone aminée non quaternisée », on entend, au sens de la présente invention, une silicone aminée ne comportant pas de groupements ammonium quaternisés.

En d'autres termes, la ou les silicones aminées utilisées dans la composition cosmétique selon la présente invention comportent dans leur structure au moins une fonction amine primaire, secondaire ou tertiaire mais ne comportent pas de fonction ammonium quaternaire.

De préférence, la ou les silicones aminées utilisées dans la composition cosmétique selon la présente invention comportent dans leur structure au moins 4 atomes de silicium.

Dans tout ce qui suit, on entend désigner par silicone, en conformité avec l'acceptation générale, tous polymères ou oligomères organosiliciés à structure linéaire ou cyclique, ramifiée ou réticulée, de poids moléculaire variable, obtenus par polymérisation et/ou par polycondensation de silanes convenablement fonctionnalisés, et constitués pour l'essentiel par une répétition de motifs principaux dans lesquels les atomes de silicium sont reliés entre eux par des atomes d'oxygène (liaison siloxane -Si-O-Si-), des radicaux hydrocarbonés éventuellement substitués, étant directement liés par l'intermédiaire d'un atome de carbone sur lesdits atomes de silicium. Les radicaux hydrocarbonés les plus courants sont les radicaux alkyles, notamment en C₁-C₁₀, et en particulier méthyle, les radicaux fluoroalkyles dont la partie alkyle est en C₁-C₁₀, les radicaux aryles et en particulier phényle.

Les silicones aminées utilisées dans la composition selon la présente invention sont choisies parmi :
(a) les composés répondant à la formule (IV) suivante :

   (R¹)ₐ(T)₃₋ₐ-Si[OSi(T)₂]ₙ-[OSi(T)_{b}(R¹)_{2-b}]ₘ-OSi(T)₃₋ₐ-(R¹)ₐ (IV)

   dans laquelle,
   T est un atome d'hydrogène, ou un radical phényle, hydroxyle (-OH), ou alkyle en C₁-C₈, et de préférence méthyle ou alcoxy en C₁-C₈, de préférence méthoxy,
   a désigne le nombre 0 ou un nombre entier de 1 à 3, et de préférence 0,
   b désigne 0 ou 1, et en particulier 1,
   m et n sont des nombres tels que la somme (n + m) peut varier notamment de 1 à 2 000 et en particulier de 50 à 150, n pouvant désigner un nombre de 0 à 1 999 et notamment de 49 à 149 et m pouvant désigner un nombre de 1 à 2 000, et notamment de 1 à 10 ;
   R₁ est un radical monovalent de formule -C_{q}H_{2q}L dans laquelle q est un nombre de 2 à 8 et L est un groupement aminé choisi parmi les groupements :

      -N(R²)-CH₂-CH₂-N(R²)₂ ;

      -N(R²)₂ ;

      dans lesquels R² peut désigner un atome d'hydrogène, un radical phényle, un radical benzyle, ou un radical hydrocarboné saturé monovalent, par exemple un radical alkyle en C₁-C₂₀.

En particulier, les silicones aminées correspondant à la définition de la formule (IV) sont choisies parmi les composés correspondant à la formule suivante (V) : 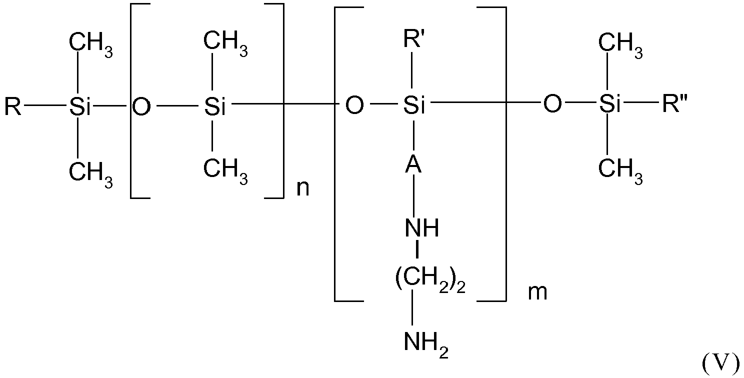 dans laquelle R, R', R", identiques ou différents, désignent un radical alkyle en C₁-C₄, de préférence CH₃ ; un radical alcoxy en C₁-C₄, de préférence méthoxy ; ou OH ; A représente un radical alkylène, linéaire ou ramifié, en C₃-C₈, de préférence en C₃-C₆; m et n sont des nombres entiers dépendant du poids moléculaire et dont la somme est comprise entre 1 et 2000.

Selon une première possibilité, R, R', R", identiques ou différents, représentent un radical alkyle en C₁-C₄ ou hydroxyle, A représente un radical alkylène en C₃ et m et n sont tels que la masse moléculaire moyenne en poids du composé est comprise entre 5 000 et 500 000 environ. Les composés de ce type sont dénommés dans le dictionnaire CTFA, "amodiméthicone".

Selon une deuxième possibilité, R, R', R", identiques ou différents, représentent un radical alcoxy en C₁-C₄ ou hydroxyle, l'un au moins des radicaux R ou R" est un radical alcoxy et A représente un radical alkylène en C₃. Le rapport molaire hydroxy / alcoxy est de préférence compris entre 0,2/1 et 0,4/1 et avantageusement égal à 0,3/1. Par ailleurs, m et n sont tels que la masse moléculaire moyenne en poids du composé est comprise entre 2000 et 10⁶. Plus particulièrement, n est compris entre 0 et 999 et m est compris entre 1 et 1000, la somme de n et m étant comprise entre 1 et 1000.

Dans cette catégorie de composés, on peut citer entre autres, le produit Belsil®ADM 652, commercialisée par Wacker.

Selon une troisième possibilité, R, R", différents, représentent un radical alcoxy en C₁-C₄ ou hydroxyle, l'un au moins des radicaux R, R" est un radical alcoxy, R' représente un radical méthyle et A représente un radical alkylène en C₃. Le rapport molaire hydroxy / alcoxy est de préférence compris entre 1/0,8 et 1/1,1, et avantageusement est égal à 1/0,95. Par ailleurs, m et n sont tels que la masse moléculaire moyenne en poids du composé est comprise entre 2000 et 200000. Plus particulièrement, n est compris entre 0 et 999 et m est compris entre 1 et 1000, la somme de n et m étant comprise entre 1 et 1000.

Plus particulièrement, on peut citer le produit FluidWR® 1300, commercialisé par Wacker.

Notons que la masse moléculaire de ces silicones est déterminée par chromatographie par perméation de gel (température ambiante, étalon polystyrène ; colonnes µ styragem ; éluant THF ; débit de 1 mm/m ; on injecte 200 µl d'une solution à 0,5 % en poids de silicone dans le THF et l'on effectue la détection par réfractométrie et UV-métrie).

Un produit correspondant à la définition de la formule (IV) est en particulier le polymère dénommé dans le dictionnaire CTFA "triméthylsilylamodiméthicone", répondant à la formule (VI) suivante: 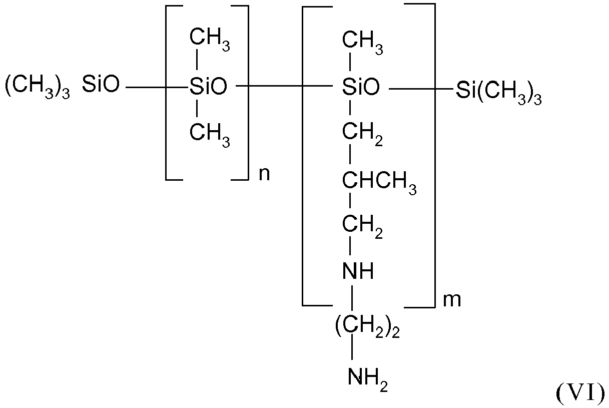 dans laquelle n et m ont les significations données ci-dessus conformément à la formule (IV).

De tels composés sont décrits par exemple dans EP 95238; un composé de formule (IV) est par exemple vendu sous la dénomination Q2-8220 par la société OSI.
b) les silicones aminées de formule (VII) : 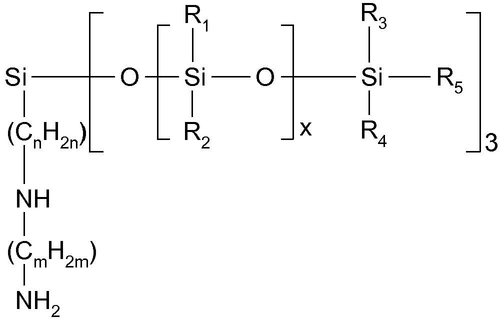 dans laquelle :
   - R₁, R₂, R₃ et R₄, identiques ou différents, désignent un radical alkyle en C₁-C₄ ou un groupement phényle,
   - R₅ désigne un radical alkyle en C₁-C₄ ou un groupement hydroxyle,
   - n est un entier variant de 1 à 5,
   - m est un entier variant de 1 à 5,
      et dans laquelle x est choisi de manière telle que l'indice d'amine soit compris entre 0,01 et 1 meq/g.

D'autres silicones aminées peuvent être utilisées dans le cadre de l'invention comme le produit référencé dans le dictionnaire CTFA sous l'appellation POLYSILICONE-9.

De préférence, la ou les silicones aminées utilisées dans la composition cosmétique selon l'invention sont choisies parmi les silicones répondant à la formule (VI).

La ou les silicones aminées utilisées dans la composition selon l'invention peuvent être présentes dans une quantité variant de 0,1 à 10% en poids, de préférence en une quantité variant de 0,2 à 5% en poids, et encore plus préférentiellement dans une quantité variant de 0,3 à 3% en poids, par rapport au poids total de la composition.

La composition cosmétique selon l'invention peut également comprendre un ou plusieurs acides organiques.

Par acide organique, on entend tout composé organique non polymérique comportant une ou plusieurs fonctions acides choisies parmi les fonctions acide carboxylique, acide sulfonique, acide phosphorique.

Préférentiellement, l'acide organique n'est pas un tensioactif.

Encore plus préférentiellement, le poids moléculaire de l'acide organique est inférieur à 250, mieux inférieur à 200.

Les acides organiques peuvent être des acides aminés.

Le ou les acides organiques sont choisis de préférence parmi l'acide acétique, l'acide propanoïque, l'acide butanoïque, l'acide lactique, l'acide malique, l'acide glycolique, l'acide ascorbique, l'acide maléïque, l'acide phtalique, l'acide succinique, la taurine, l'acide tartrique, l'arginine, la glycine, l'acide glucuronique, l'acide gluconique et l'acide citrique.

Encore plus préférentiellement, les acides organiques selon l'invention sont les acides carboxyliques et mieux les acides carboxyliques alpha-hydroxylés ou AHA.

Encore plus préférentiellement encore, l'acide organique utilisé dans la composition selon l'invention est l'acide lactique et l'acide citrique et de préférence l'acide lactique.

Dans la composition, l'acide organique peut être sous forme libre ou salifiée.

Le ou les acides organiques pouvant être utilisés dans la composition selon la présente invention peuvent être présents en une teneur exprimée en acides libres allant de 0,01 à 10% en poids, de préférence en une teneur allant de 0,1 à 8% en poids, et encore plus préférentiellement en une teneur allant de 0,2 à 5% en poids, par rapport au poids total de la composition.

La composition selon la présente invention peut comprendre également un ou plusieurs tensioactifs additionnels choisis parmi les tensioactifs amphotères et non ioniques.

Les agents tensioactifs amphotères ou zwittérioniques, utilisables dans la présente invention, peuvent être notamment des dérivés d'amines aliphatiques secondaires ou tertiaires, dans lesquels le groupe aliphatique est une chaîne linéaire ou ramifiée comportant de 8 à 22 atomes de carbone et contenant au moins un groupe anionique tel que, par exemple, un groupe carboxylate, sulfonate, sulfate, phosphate ou phosphonate. On peut citer en particulier les alkyl(C₈₋₂₀)bétaïnes, les sulfobétaïnes, les (alkyl en C₈₋₂₀)amido(alkyl en C₆₋₈)bétaïnes ou les (alkyl en C₈₋₂₀)amido(alkyl en C₆₋₈)sulfobétaïnes.

Parmi les dérivés d'amines, on peut citer les produits commercialisés sous la dénomination MIRANOL^{®}, tels que décrits dans les brevets US 2 528 378 et US 2 781 354 et classés dans le dictionnaire CTFA, 3ème édition, 1982, sous les dénominations Amphocarboxy-glycinate et Amphocarboxypropionate de structures respectives (VIII) et (IX) :

Rₐ-CONHCH₂CH₂-N(R_{b})(R_{c})(CH₂COO⁻) (VIII)

dans laquelle:
Rₐ représente un groupe alkyle dérivé d'un acide Rₐ-COOH présent dans l'huile de coprah hydrolysée, un groupe heptyle, nonyle ou undécyle,
R_{b} représente un groupe bêta-hydroxyéthyle, et
R_{c} représente un groupe carboxyméthyle ;
   et

   Rₐ'-CONHCH₂CH₂-N(B)(B') (IX)

   dans laquelle :
   B représente -CH₂CH₂OX',
   B' représente -(CH₂)_{z}-Y', avec z = 1 ou 2,
   X' représente le groupe -CH₂CH₂-COOH ou un atome d'hydrogène,
   Y' représente -COOH ou le groupe -CH₂-CHOH-SO₃H,
   Rₐ' représente un groupe alkyle d'un acide Rₐ'-COOH présent dans l'huile de coprah ou dans l'huile de lin hydrolysée, un groupe alkyle, notamment en C₁₇ et sa forme iso, un groupe en C₁₇ insaturé.

Ces composés sont classés dans le dictionnaire CTFA, 5ème édition, 1993, sous les dénominations cocoamphodiacétate de disodium, lauroamphodiacétate de disodium, caprylamphodiacétate de disodium, capryloamphodiacétate de disodium, cocoamphodipropionate de disodium, lauroamphodipropionate de disodium, caprylamphodipropionate de disodium, capryloamphodipropionate de disodium, acide lauroamphodipropionique, acide cocoamphodipropionique.

A titre d'exemple, on peut citer le cocoamphodiacétate commercialisé par la société RHODIA sous la dénomination commerciale MIRANOL^{®} C2M concentré.

Parmi les tensioactifs amphotères ou zwittérioniques cités ci-dessus, on utilise de préférence les (alkyl en C₈₋₂₀)-bétaïnes, les (alkyl en C₈₋₂₀)-amido(alkyl en C₆₋₈)bétaïnes et leurs mélanges.

Lorsqu'ils sont présents, la quantité du ou des tensioactifs amphotères ou zwittérioniques varie de préférence dans l'intervalle allant de 0,1 à 15 % en poids, mieux encore de 0,5 à 10% en poids, encore mieux de 1 à 8% en poids, par rapport au poids total de la composition.

Des exemples de tensioactifs non-ioniques additionnels utilisables dans les compositions de la présente invention sont décrits par exemple dans "Handbook of Surfactants" par M.R. PORTER, éditions Blackie & Son (Glasgow and London), 1991, pp 116-178. Ils sont choisis notamment parmi les alcools, les alpha-diols, les alkyl(C₁₋₂₀)phénols ou les acides gras polyéthoxylés, polypropoxylés ou polyglycérolés, ayant une chaîne grasse comportant, par exemple, de 8 à 18 atomes de carbone, le nombre de groupements oxyde d'éthylène ou oxyde de propylène pouvant aller notamment de 2 à 50 et le nombre de groupements glycérol pouvant aller notamment de 2 à 30.

On peut également citer les condensats d'oxyde d'éthylène et d'oxyde de propylène sur des alcools gras ; les amides gras polyéthoxylés ayant de préférence de 2 à 30 motifs d'oxyde d'éthylène, les amides gras polyglycérolés comportant en moyenne de 1 à 5 groupements glycérol et en particulier de 1,5 à 4, les esters d'acides gras du sorbitan éthoxylés ayant de 2 à 30 motifs d'oxyde d'éthylène, les esters d'acides gras du saccharose, les esters d'acides gras du polyéthylèneglycol, les (alkyl en C₆₋₂₄)polyglycosides, les dérivés de N-(alkyl en C₆₋₂₄)glucamine, les oxydes d'amines tels que les oxydes d'(alkyl en C₁₀₋₁₄)amines ou les oxydes de N-(acyl en C₁₀₋₁₄)-aminopropylmorpholine.

Lorsqu'ils sont présents, la quantité du ou des tensioactifs non ioniques additionnels varie de préférence de 0,01 à 20% en poids, mieux encore de 0,1 à 10 % en poids par rapport au poids total de la composition.

De préférence, la quantité totale en tensioactifs dans la composition cosmétique selon l'invention varie de 3 à 50% en poids, plus préférentiellement de 5 à 30% en poids, mieux de 8 à 20% en poids, par rapport au poids total de la composition cosmétique.

La composition cosmétique peut également comprendre un ou plusieurs polymères cationiques.

Au sens de la présente invention, l'expression "polymère cationique" désigne tout polymère contenant des groupements cationiques et/ou des groupements ionisables en groupements cationiques.

Les polymères cationiques éventuellement présents dans la composition selon l'invention peuvent être choisis parmi tous ceux déjà connus en soi comme améliorant les propriétés cosmétiques des cheveux, à savoir notamment ceux décrits dans la demande de brevet EP-A-337 354 et dans les brevets français FR-2 270 846, 2 383 660, 2 598 611, 2 470 596 et 2 519 863.

Les polymères cationiques préférés sont choisis parmi ceux qui contiennent des motifs comportant des groupements amine primaire, secondaire, tertiaire et/ou quaternaire pouvant, soit faire partie de la chaîne principale polymère, soit être portés par un substituant latéral directement relié à celle-ci.

Les polymères cationiques utilisés ont généralement une masse moléculaire moyenne en nombre comprise entre 500 et 5.10⁶ environ, et de préférence comprise entre 10³ et 3.10⁶ environ.

Parmi les polymères cationiques, on peut citer plus particulièrement les polymères du type polyamine, polyaminoamide et polyammonium quaternaire.

Ce sont des produits connus. Ils sont notamment décrits dans les brevets français n° 2 505 348 ou 2 542 997. Parmi lesdits polymères, on peut citer :
(1) Les homopolymères ou copolymères dérivés d'esters ou d'amides acryliques ou méthacryliques et comportant au moins un des motifs de formules (X), (XI), (XII) ou (XIII) suivantes: 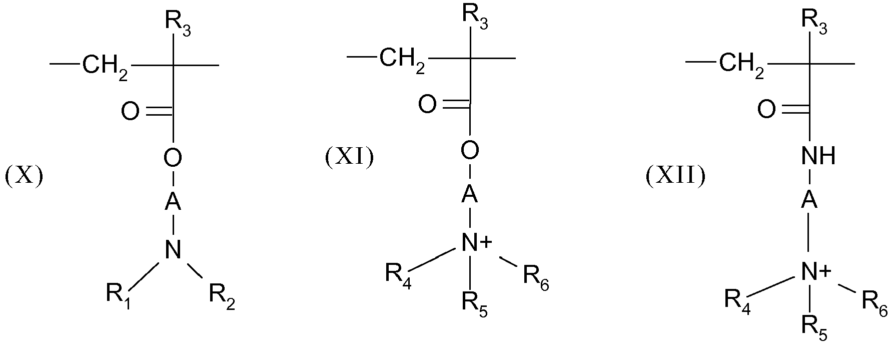 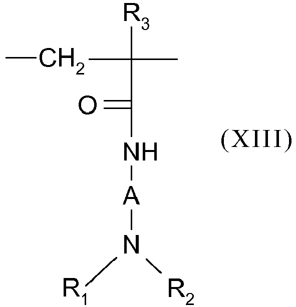 dans lesquelles:
   R₃, identiques ou différents, désignent un atome d'hydrogène ou un radical CH₃;
   A, identiques ou différents, représentent un groupe alkyle, linéaire ou ramifié, de 1 à 6 atomes de carbone, de préférence 2 ou 3 atomes de carbone ou un groupe hydroxyalkyle de 1 à 4 atomes de carbone ;
   R₄, R₅, R₆, identiques ou différents, représentent un groupe alkyle ayant de 1 à 18 atomes de carbone ou un radical benzyle et de préférence un groupe alkyle ayant de 1 à 6 atomes de carbone;
   R₁ et R₂, identiques ou différents, représentent hydrogène ou un groupe alkyle ayant de 1 à 6 atomes de carbone et de préférence méthyle ou éthyle;
   X désigne un anion dérivé d'un acide minéral ou organique tel qu'un anion méthosulfate ou un halogénure tel que chlorure ou bromure.

On peut citer en particulier l'homopolymère chlorure de méthacrylate d'éthyl triméthyl ammonium.

Les polymères de la famille (1) peuvent contenir en outre un ou plusieurs motifs dérivant de comonomères pouvant être choisis dans la famille des acrylamides, méthacrylamides, diacétones acrylamides, acrylamides et méthacrylamides substitués sur l'azote par des alkyles inférieurs (C₁-C₄), des acides acryliques ou méthacryliques ou leurs esters, des vinyllactames tels que la vinylpyrrolidone ou le vinylcaprolactame, des esters vinyliques.

Ainsi, parmi ces polymères de la famille (1), on peut citer :
- les copolymères d'acrylamide et de diméthylaminoéthyl méthacrylate quaternisé au sulfate de diméthyle ou avec un hologénure de diméthyle, tel que celui vendu sous la dénomination HERCOFLOC par la société HERCULES,
- les copolymères d'acrylamide et de chlorure de méthacryloyloxyéthyltriméthylammonium décrits par exemple dans la demande de brevet EP-A-080976 et vendus sous la dénomination BINA QUAT P 100 par la société CIBA GEIGY,
- le copolymère d'acrylamide et de méthosulfate de méthacryloyloxyéthyltriméthylammonium vendu sous la dénomination RETEN par la société HERCULES,
- les copolymères vinylpyrrolidone / acrylate ou méthacrylate de dialkylaminoalkyle quaternisés ou non, tels que les produits vendus sous la dénomination "GAFQUAT" par la société ISP comme par exemple "GAFQUAT 734" ou "GAFQUAT 755" ou bien les produits dénommés "COPOLYMER 845, 958 et 937". Ces polymères sont décrits en détail dans les brevets français 2.077.143 et 2.393.573,
- les terpolymères méthacrylate de diméthyl amino éthyle/ vinylcaprolactame/ vinylpyrrolidone tel que le produit vendu sous la dénomination GAFFIX VC 713 par la société ISP,
- les copolymère vinylpyrrolidone / méthacrylamidopropyl dimethylamine commercialisés notamment sous la dénomination STYLEZE CC 10 par ISP,
- les copolymères vinylpyrrolidone / méthacrylamide de diméthylaminopropyle quaternisés tel que le produit vendu sous la dénomination "GAFQUAT HS 100" par la société ISP,
- les polymères réticulés de sels de méthacryloyloxyalkyl(C₁-C₄) trialkyl(C₁-C₄)ammonium tels que les polymères obtenus par homopolymérisation du diméthylaminoéthylméthacrylate quaternisé par le chlorure de méthyle, ou par copolymérisation de l'acrylamide avec le diméthylaminoéthylméthacrylate quaternisé par le chlorure de méthyle, l'homo ou la copolymérisation étant suivie d'une réticulation par un composé à insaturation oléfinique, en particulier le méthylène bis acrylamide. On peut plus particulièrement utiliser un copolymère réticulé acrylamide/chlorure de méthacryloyloxyéthyl triméthylammonium (20/80 en poids) sous forme de dispersion contenant 50 % en poids dudit copolymère dans de l'huile minérale. Cette dispersion est commercialisée sous le nom de " SALCARE^{®} SC 92 " par la Société CIBA. On peut également utiliser un homopolymère réticulé du chlorure de méthacryloyloxyéthyl triméthylammonium contenant environ 50 % en poids de l'homopolymère dans de l'huile minérale ou dans un ester liquide. Ces dispersions sont commercialisées sous les noms de " SALCARE^{®} SC 95 " et " SALCARE^{®} SC 96 " par la Société CIBA.

(2) Les dérivés d'éthers de cellulose comportant des groupements ammonium quaternaire décrits dans le brevet français 1 492 597, et en particulier les polymères commercialisés sous les dénominations "UCARE POLYMER JR" (JR 400, JR 125, JR 30M) ou "LR" (LR 400, LR 30M) par la société AMERCHOL. Ces polymères sont également définis dans le dictionnaire CTFA comme des ammoniums quaternaires d'hydroxyéthylcellulose ayant réagi avec un époxyde substitué par un groupement triméthylammonium.
(3) Les copolymères de cellulose ou les dérivés de cellulose greffés avec un monomère hydrosoluble d'ammonium quaternaire, et décrits notamment dans le brevet US 4 131 576, tels que les hydroxyalkyl celluloses, comme les hydroxyméthyl-, hydroxyéthyl- ou hydroxypropyl celluloses greffées notamment avec un sel de méthacryloyléthyl triméthylammonium, de méthacrylmidopropyl triméthylammonium, de diméthyl-diallylammonium.
   Les produits commercialisés répondant à cette définition sont plus particulièrement les produits vendus sous la dénomination "Celquat L 200" et "Celquat H 100" par la Société National Starch.
(4) Les gommes de guar cationiques décrits plus particulièrement dans les brevets US 3 589 578 et 4 031 307 tel que les gommes de guar contenant des groupements cationiques trialkylammonium. On utilise par exemple des gommes de guar modifiées par un sel (par ex. chlorure) de 2,3-époxypropyl triméthylammonium.
   De tels produits sont commercialisés notamment sous les dénominations commerciales de JAGUAR C13 S, JAGUAR C 15, JAGUAR C 17 ou JAGUAR C162 par la société RHODIA.
(5) Les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets français 2.162.025 et 2.280.361.
(6) Les polyaminoamides solubles dans l'eau préparés en particulier par polycondensation d'un composé acide avec une polyamine ; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé ; l'agent réticulant étant utilisé dans des proportions allant de 0,025 à 0,35 mole par groupement amine du polyaminoamide ; ces polyaminoamides peuvent être alcoylés ou s'ils comportent une ou plusieurs fonctions amines tertiaires, quaternisées. De tels polymères sont notamment décrits dans les brevets français 2.252.840 et 2.368.508.
(7) Les dérivés de polyaminoamides résultant de la condensation de polyalcoylènes polyamines avec des acides polycarboxyliques suivie d'une alcoylation par des agents bifonctionnels. On peut citer par exemple les polymères acide adipique-diacoylaminohydroxyalcoyldialoylène triamine dans lesquels le radical alcoyle comporte de 1 à 4 atomes de carbone et désigne de préférence méthyle, éthyle, propyle. De tels polymères sont notamment décrits dans le brevet français 1.583.363.
   Parmi ces dérivés, on peut citer plus particulièrement les polymères acide adipique/diméthylaminohydroxypropyl/diéthylène triamine vendus sous la dénomination "Cartaretine F, F4 ou F8" par la société Sandoz.
(8) Les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone. Le rapport molaire entre le polyalkylène polylamine et l'acide dicarboxylique étant compris entre 0,8 : 1 et 1,4 : 1; le polyaminoamide en résultant étant amené à réagir avec l'épichlorhydrine dans un rapport molaire d'épichlorhydrine par rapport au groupement amine secondaire du polyaminoamide compris entre 0,5 : 1 et 1,8 : 1. De tels polymères sont notamment décrits dans les brevets américains 3.227.615 et 2.961.347.
   Des polymères de ce type sont en particulier commercialisés sous la dénomination "Hercosett 57" par la société Hercules Inc. ou bien sous la dénomination de "PD 170" ou "Delsette 101" par la société Hercules dans le cas du copolymère d'acide adipique/époxypropyl/diéthylène-triamine.
(9) Les cyclopolymères d'alkyl diallyl amine ou de dialkyl diallyl ammonium tels que les homopolymères ou copolymères comportant comme constituant principal de la chaîne des motifs répondant aux formules (XIV) ou (XV) : 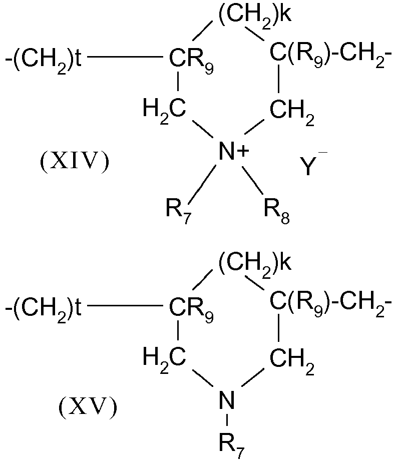 formules dans lesquelles k et t sont égaux à 0 ou 1, la somme k + t étant égale à 1 ; R₉ désigne un atome d'hydrogène ou un radical méthyle ; R₇ et R₈, indépendamment l'un de l'autre, désignent un groupement alkyle ayant de 1 à 6 atomes de carbone, un groupement hydroxyalkyle dans lequel le groupement alkyle a de préférence 1 à 5 atomes de carbone, un groupement amidoalkyle inférieur (C₁-C₄), ou R₇ et R₈ peuvent désigner conjointement avec l'atome d'azote auquel ils sont rattachés, des groupement hétérocycliques, tels que pipéridinyle ou morpholinyle ; R₇ et R₈ indépendamment l'un de l'autre désignent de préférence un groupement alkyle ayant de 1 à 4 atomes de carbone ; Y⁻ est un anion tel que bromure, chlorure, acétate, borate, citrate, tartrate, bisulfate, bisulfite, sulfate, phosphate. Ces polymères sont notamment décrits dans le brevet français 2.080.759 et dans son certificat d'addition 2.190.406.
   Parmi les polymères définis ci-dessus, on peut citer plus particulièrement l'homopolymère de chlorure de diméthyldiallylammonium vendu sous la dénomination "Merquat 100" par la société NALCO (et ses homologues de faible masse moléculaire moyenne en poids) et les copolymères de chlorure de diallyldiméthylammonium et d'acrylamide commercialisés sous la dénomination "MERQUAT 550", "MERQUAT 7SPR".
(10) Le polymère de diammonium quaternaire contenant des motifs récurrents répondant à la formule : 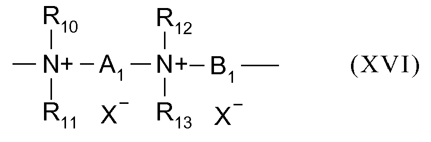 formule (XVI) dans laquelle :
   R₁₀, R₁₁, R₁₂ et R_{13,} identiques ou différents, représentent des radicaux aliphatiques, alicycliques, ou arylaliphatiques contenant de 1 à 6 atomes de carbone ou des radicaux hydroxyalkylaliphatiques inférieurs, ou bien R₁₀, R₁₁, R₁₂ et R₁₃, ensemble ou séparément, constituent avec les atomes d'azote auxquels ils sont rattachés des hétérocycles contenant éventuellement un second hétéroatome autre que l'azote ou bien R₁₀, R₁₁, R₁₂ et R₁₃ représentent un radical alkyle en C₁-C₆ linéaire ou ramifié substitué par un groupement nitrile, ester, acyle, amide ou -CO-O- R₁₄-D ou -CO-NH- R₁₄-D où R₁₄ est un alkylène et D un groupement ammonium quaternaire ;
   A1 et B1 représentent des groupements polyméthyléniques contenant de 2 à 8 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
   X⁻ désigne un anion dérivé d'un acide minéral ou organique;
   A1, R₁₀ et R₁₂ peuvent former avec les deux atomes d'azote auxquels ils sont rattachés un cycle pipérazinique ; en outre si A1 désigne un radical alkylène ou hydroxyalkylène linéaire ou ramifié, saturé ou insaturé, B1 peut également désigner un groupement-(CH₂)n-CO-D-OC-(CH₂)n- dans lequel D désigne :
      a) un reste de glycol de formule : -O-Z-O-, où Z désigne un radical hydrocarboné linéaire ou ramifié ou un groupement répondant à l'une des formules suivantes :

         -(CH₂-CH₂-O)x-CH₂-CH₂-

         -[CH₂-CH(CH₃)-O]y-CH₂-CH(CH₃)-

         où x et y désignent un nombre entier de 1 à 4, représentant un degré de polymérisation défini et unique ou un nombre quelconque de 1 à 4 représentant un degré de polymérisation moyen ;
      b) un reste de diamine bis-secondaire tel qu'un dérivé de pipérazine ;
      c) un reste de diamine bis-primaire de formule : -NH-Y-NH-, où Y désigne un radical hydrocarboné linéaire ou ramifié, ou bien le radical bivalent

         -CH₂-CH₂-S-S-CH₂-CH₂- ;
      d) un groupement uréylène de formule : -NH-CO-NH- .

   De préférence, X⁻ est un anion tel que le chlorure ou le bromure.
   Ces polymères ont une masse moléculaire moyenne en nombre généralement comprise entre 1000 et 100000.
   Des polymères de ce type sont notamment décrits dans les brevets français 2.320.330, 2.270.846, 2.316.271, 2.336.434 et 2.413.907 et les brevets US 2.273.780, 2.375.853, 2.388.614, 2.454.547, 3.206.462, 2.261.002, 2.271.378, 3.874.870, 4.001.432, 3.929.990, 3.966.904, 4.005.193, 4.025.617, 4.025.627, 4.025.653, 4.026.945 et 4.027.020.
   On peut utiliser plus particulièrement les polymères qui sont constitués de motifs récurrents répondant à la formule (XVI) suivante: 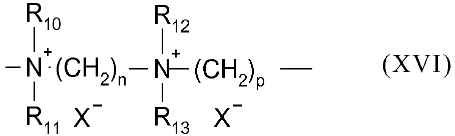 dans laquelle R₁₀, R₁₁, R₁₂ et R₁₃, identiques ou différents, désignent un radical alkyle ou hydroxyalkyle ayant de 1 à 4 atomes de carbone environ, n et p sont des nombres entiers variant de 2 à 8 environ et, X⁻ est un anion dérivé d'un acide minéral ou organique. On peut citer en particulier le MEXOMERE PO commercialisé par la société CHIMEX.
(11) Les polyammoniums quaternaires constitués de motifs récurrents de formule (XVII) : 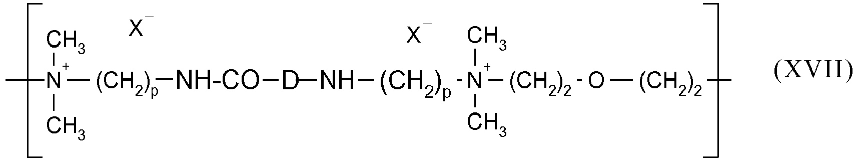 dans laquelle p désigne un nombre entier variant de 1 à 6 environ, D peut être nul ou peut représenter un groupement -(CH₂)ᵣ-CO- dans lequel r désigne un nombre égal à 4 ou à 7, X⁻ est un anion ;
   De tels polymères peuvent être préparés selon les procédés décrits dans les brevets U.S.A. n° 4 157 388, 4 702 906, 4 719 282. Ils sont notamment décrits dans la demande de brevet EP-A-122 324.
   Parmi eux, on peut par exemple citer, les produits "Mirapol A 15", "Mirapol AD1", "Mirapol AZ1" et "Mirapol 175" vendus par la société Miranol.
(12) Les polymères quaternaires de vinylpyrrolidone et de vinylimidazole tels que par exemple les produits commercialisés sous les dénominations Luviquat FC 905, FC 550 et FC 370 par la société B.A.S.F. Ces polymères peuvent également comprendre d'autres monomères comme les halogénures de diallyldialkylammonium. On peut citer, en particulier, le produit commercialisé sous la dénomination Luviquat Sensation par la société B.A.S.F.
(13) Les polyamines comme le Polyquart H vendu par HENKEL, référencé sous le nom de " POLYETHYLENEGLYCOL (15) TALLOW POLYAMINE " dans le dictionnaire CTFA, ou les polyamines de coprah oxyéthyléné (15 OE).

D'autres polymères cationiques utilisables dans le cadre de l'invention sont des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.

Parmi tous les polymères cationiques susceptibles d'être utilisés dans le cadre de la présente invention, on préfère mettre en oeuvre les polymères des familles (1), (2), (3), (4), (9), (10) et (12).

De préférence, le ou les polymères cationiques sont choisi parmi les celluloses cationiques et les gommes guar cationiques.

De préférence, le ou les polymères cationiques sont choisis parmi les celluloses cationiques, les gommes de guar cationiques et les polymères quaternaires de vinylpyrrolidone et de vinyl imidazole éventuellement associé à d'autres monomères.

Plus préférentiellement, le ou les polymères cationiques sont choisis parmi les hydroxyalkylcelluloses, comme les hydroxyméthyl-, hydroxyéthyl- ou hydroxypropyl celluloses greffées notamment avec un sel de méthacryloyléthyl triméthylammonium, de méthacrylamidopropyltriméthylammonium, de diméthyldiallyl ammonium, les gommes de guar cationiques le copolymère vinylpyrrolidone et de vinylimidazole et chlorure de diméthyldiallylammonium.

La teneur en polymère(s) cationique(s) dans la composition selon l'invention peut varier de 0,01 à 5% en poids par rapport au poids total de la composition, de préférence de 0,1 à 1% en poids, et plus préférentiellement encore de 0,15 à 0,5% en poids.

Par milieu cosmétiquement acceptable, on entend un milieu compatible avec les fibres kératiniques, telles que les cheveux.

Le milieu cosmétiquement acceptable est constitué d'eau ou d'un mélange d'eau et d'un ou plusieurs solvants cosmétiquement acceptables choisis parmi les alcools inférieurs en C₁-C₄, tels que l'éthanol, l'isopropanol, le tertio-butanol ou le n-butanol ; les polyols tels que le glycérol, le propylèneglycol et les polyéthylèneglycols ; et leurs mélanges.

Le pH des compositions selon l'invention varie généralement de 3 à 11 et de préférence de 5 à 10, mieux de 7 à 10.

La composition selon l'invention peut comprendre en outre un ou plusieurs additifs classiques bien connus dans la technique, tels que; des épaississants ou régulateurs de viscosité, naturels ou synthétiques ; des alcools gras en C₁₂-C₃₀ ; des céramides ; des esters gras huileux tels que le myristate d'isopropyle, le myristate de myristyle, le palmitate de cétyle et le stéarate de stéaryle ; des huiles minérales, végétales ou synthétiques telles que les α-oléfines ou l'huile de palme ; des vitamines ou provitamines ; des polymères amphotères ; des agents de stabilisation du pH, des conservateurs, des silicones autres que les silicones aminées de l'invention et des colorants.

Le ou les agents épaississants peuvent être choisis parmi les agents épaississants cellulosiques, par exemple l'hydroxyéthylcellulose, l'hydroxypropylcellulose et le carboxyméthylcellulose, la gomme de guar et ses dérivés, par exemple l'hydroxypropyl guar, commercialisé par la société RHODIA sous la référence JAGUAR HP 105, les gommes d'origine microbienne, telle que la gomme de xanthane et la gomme de scléroglucane, les agents épaississants synthétiques tels que les homopolymères réticulés d'acide acrylique ou d'acide acrylamidopropanesulfonique, par exemple le Carbomer, les polymères associatifs non ioniques, anioniques, cationiques ou amphotères, tels que les polymères commercialisés sous les appellations PEMULEN TR1 ou TR2 par la société GOODRICH, SALCARE SC90 par la société CIBA, ACULYN 22, 28, 33, 44 ou 46 par la société ROHM & HAAS et ELFACOS T210 et T212 par la société AKZO.

L'homme de métier veillera à choisir les éventuels additifs et leur quantité de manière à ce qu'ils ne nuisent pas aux propriétés des compositions de la présente invention.

Ces additifs sont généralement présents dans la composition selon l'invention en une quantité allant de 0 à 20 % en poids par rapport au poids total de la composition.

De préférence, les compositions cosmétiques de l'invention sont transparentes ou translucides, c'est-à-dire que ces compositions présentent une transmittance à 600 nanomètres supérieure à 85%, mieux supérieure à 90% et encore mieux supérieure à 94%.

Les compositions conformes à l'invention peuvent être utilisées comme shampooings pour le lavage et le conditionnement des cheveux et ils sont appliqués dans ce cas-là, de préférence, sur les cheveux généralement humides dans des quantités efficaces pour les laver, et la mousse générée par massage ou friction avec les mains peut être ensuite éliminée après un éventuel temps de pause, par rinçage avec de l'eau, l'opération pouvant être répétée une ou plusieurs fois.

Ainsi la présente invention concerne également l'utilisation d'une composition cosmétique telle que décrite ci-avant, comme shampooing, pour le nettoyage et le conditionnement des fibres kératiniques, en particulier des fibres kératiniques humaines telles que les cheveux.

Un autre objet de l'invention est un procédé de traitement cosmétique des fibres kératiniques, telles que les cheveux, qui consiste à appliquer une quantité efficace d'une composition telle que décrite ci-dessus, sur lesdites fibres, et de préférence à rincer après un éventuel temps de pose.

En particulier, le procédé de traitement cosmétique des fibres kératiniques est un procédé de lavage et de conditionnement des fibres kératiniques, en particulier des cheveux.

Les exemples suivants servent à illustrer la présente invention.

### EXEMPLE

### Exemple 1 :

On prépare la composition (A) selon l'invention à partir des ingrédients indiqués dans le tableau ci-dessous dont les quantités sont exprimées en pour cent en poids en produit en l'état, par rapport au poids total de la composition.

| Composition | A (invention) |
|---|---|
| Acide lactique | 0,27 |
| Mélange de chloro-5-méthyl-2-iosthiazoline4-one-3 / méthylisothiazoline-4-one-3 / chlorure et nitrate de magnésium ⁽¹⁾ | 0,1 |
| Oléate de propylène glycol polyéthoxylé (55 OE) et de propylène glycol en solution hydroglycolique ⁽²⁾ | 0,6 |
| Hydroxyéthyl cellulose quaternisée par le chlorure de 2,3 époxypropyl triméthyl ammonium à 95,5 % MA ⁽³⁾ | 0,6 |
| Polydiméthyl siloxane à groupements aminoéthyl aminoisobutyle et triméthylsiloxy ⁽⁴⁾ | 1 |
| 3-aminopropyltriéthoxysilane ⁽⁵⁾ | 0,75 |
| Cocoyl bétaïne en solution aqueuse à 30 %MA (6) | 17 |
| Alcool cétylique oxyéthyléné (20 OE) et oxypropyléné (5 OP) ⁽⁷⁾ | 0,5 |
| Acide lauryl éther carboxylique (4,5 OE) à 90% MA ⁽⁸⁾ | 1 |
| Monoisopropanolamide d'acides de coprah ⁽⁹⁾ | 0,85 |
| Lauryléther sulfate de sodium (2.2 OE) en solution aqueuse (70% M.A) ⁽¹⁰⁾ | 16 |
| Agent de mise au pH | qs pH=9 |
| Parfum | 0,5 |
| Eau désionisée | qsp 100 g |

| | |
|---|---|
| ⁽¹⁾ vendu sous la dénomination commerciale KATHON CG par la société ROHM et HAAS ⁽²⁾ vendu sous la dénomination commerciale ANTIL 141 LIQUID par la société EVONIK GOLDSCHMIDT, ⁽³⁾ vendu sous la dénomination commerciale POLYQUTA 400 KC par la société KCI, ⁽⁴⁾ vendu sous la dénomination commerciale DC2 8566 AMINOFLUID par la société DOW CORNING, ⁽⁵⁾ vendu sous la dénomination XIAMETER OFS 6011 SILANE par la société DOW CORNING, ⁽⁶⁾ vendu sous la dénomination MIRATAINE BB/FLA par la société RHODIA, ⁽⁷⁾ vendu sous la dénomination PROCETYL AWS-LQ par la société CRODA, ⁽⁸⁾ vendu sous la dénomination AKYPO RLM 45 CA par la société KAO, ⁽⁹⁾ vendu sous la dénomination EMPILAN CIS par la société HUNTSMAN, ⁽¹⁰⁾ vendu sous la dénomination TEXAPON AOS 225UP par la société COGNIS | |

On obtient une composition qui est limpide et stable dans le temps.

Appliquée en tant que shampooing, la composition (A) confère aux cheveux de la masse, du volume ainsi qu'un toucher doux satisfaisant.

### Exemple 2 :

On prépare la composition (B) selon l'invention à partir des ingrédients indiqués dans le tableau ci-dessous dont les quantités sont exprimées en pour cent en poids en matières actives, par rapport au poids total de la composition.

| Composition | B |
|---|---|
| Chlorure de sodium | 1,436 |
| Acide lactique | 0,2 |
| Distéarate d'éthylène glycol | 1,6 |
| Chlorure d'hydroxypropylguar triméthyl ammonium ⁽¹⁾ | 0,2 |
| Polymère carboxyvinylique ⁽²⁾ | 0,15 |
| Polydiméthylsiloxane à groupements aminoéthyl iminopropyl à fonction méthoxy et/ou hydroxy et alpha-oméga silanols en émulsion aqueuse cationique à 60% ⁽³⁾ | 1,7 |
| Hexylène glycol | 0,5 |
| 3-aminopropyltriéthoxysilane ⁽⁷⁾ | 0,75 |
| Cocoyl bétaïne en solution aqueuse à 30 % MA ⁽⁵⁾ | 6 |
| Monoisopropanolamide d'acides de coprah ⁽⁶⁾ | 0,7 |
| Lauryléther sulfate de sodium (2.2 OE) en solution aqueuse (70% M.A) ⁽⁷⁾ | 17,4 |
| Agent de mise au pH | qs pH = 9 |
| Parfum | 0,5 |
| Eau désionisée | qsp 100 g |

| | |
|---|---|
| ⁽¹⁾ vendu sous la dénomination commerciale JAGUAR C13S par la société RHODIA ⁽²⁾ vendu sous la dénomination commerciale CARBOPOL 980 POLYMER par la société LUBRIZOL, ⁽³⁾ vendu sous la dénomination commerciale DC2-8299 CATIONIC EMULSION par la société DOW CORNING, ⁽⁴⁾ vendu sous la dénomination commerciale XIAMETER OFS 6011 SILANE par la société DOW CORNING, ⁽⁵⁾ vendu sous la dénomination MIRATAINE BB/FLA par la société RHODIA, ⁽⁶⁾ vendu sous la dénomination EMPILAN CIS par la société HUNTSMAN ⁽⁷⁾ vendu sous la dénomination Texapon AOS 225UP par la société COGNIS | |

On obtient également une composition nacrée qui est stable dans le temps.

Appliquée en tant que shampooing, la composition (B) confère aux cheveux de la masse, du volume ainsi qu'un toucher doux satisfaisant.

## Revendications

1. Composition cosmétique de nettoyage et de conditionnement des fibres kératiniques, en particulier des fibres kératiniques humaines telles que les cheveux, comprenant dans un milieu cosmétiquement acceptable :
i) un ou plusieurs composés organiques du silicium choisis parmi les silanes comprenant un atome de silicium et les siloxanes comprenant deux ou trois atomes de silicium, lesdits composés organiques du silicium comportant en outre une ou plusieurs fonctions chimiques basiques et un ou plusieurs groupes hydroxyles ou hydrolysables par molécule ;
ii) un ou plusieurs tensioactifs anioniques ; et
iii) une ou plusieurs silicones aminées non quaternisées différentes des composés i).

2. Composition cosmétique selon la revendication 1, **caractérisée en ce que** les fonctions chimiques basiques du composé organique du silicium sont choisies parmi les amines primaires, secondaires ou tertiaires.

3. Composition cosmétique selon la revendication 1 ou 2, **caractérisée en ce que** les groupes hydrolysables sont choisis parmi les groupes alcoxy, aryloxy et halogène.

4. Composition cosmétique selon l'une quelconque des revendications 1 à 3, **caractérisée en ce que** le ou les composés organiques du silicium sont choisis parmi les composés de formule (I) : 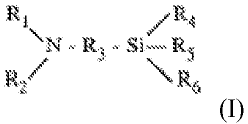 dans laquelle :
R₄ représente un halogène, un groupe OR' ou R'₁ ;
R₅ représente un halogène, un groupe OR" ou R'₂ ;
R₆ représente un halogène, un groupe OR'" ou R'₃ ;
R₁, R₂, R₃, R', R", R"', R'₁, R'₂, R'₃ représentent, indépendamment les uns des autres, un groupe hydrocarboné saturé ou insaturé, linéaire ou ramifié, portant éventuellement des groupes chimiques supplémentaires, R₁, R₂, R', R" et R'" pouvant en outre désigner l'hydrogène, et deux au moins des groupes R₄, R₅ et R₆ désignant respectivement OR', OR" et OR"', deux au moins des groupes R',
R" et R"' étant différents de l'hydrogène ; et 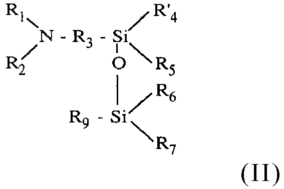 dans laquelle :
R₁, R₂, R₃, R₅ et R₆ sont définis comme précédemment ;
R'₄ représente un atome d'halogène ou un groupe OR₁₁ ;
R₇ représente un atome d'halogène, un groupe OR₁₀ ou R"₁ ;
R₉ représente un atome d'halogène, un groupe OR₈, R"₂ ou R₃NR₁R₂ ; R"₁, R"₂, R₈, R₁₀ et R₁₁ représente un groupe hydrocarboné, saturé ou insaturé, linéaire ou ramifié, portant éventuellement des groupes chimiques supplémentaires, les groupes R₁, R₁₀ et R₈ pouvant en outre représenter un atome d'hydrogène ; l'un au moins des groupes R₆, R₇ et R₉ désignant un atome d'halogène, un groupe OR"', OR₁₀ ou OR₈.

5. Composition cosmétique selon la revendication 4, **caractérisée en ce que** les groupes R₁, R₂, R', R'₁, R'₂, R'₃, R", R"', R"₁, R"₂, R₈, R₁₀ et R₁₁ sont choisis parmi les radicaux alkyle de C₁-C₁₂, aryle de C₆ à C₁₄, alkyle de C₁ à C₈-aryle de C₆ à C₁₄, et aryle de C₆ à C₁₄-alkyle de C₁ à C₈.

6. Composition cosmétique selon l'une quelconque des revendications précédentes, **caractérisée en ce que** le ou les composés organiques du silicium sont choisis parmi les composés de formule (III) : 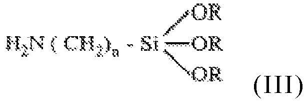 dans laquelle les radicaux R, identiques ou différents, sont choisis parmi les radicaux alkyle en C₁-C₆ et n est un nombre entier de 1 à 6, de préférence de 2 à 4.

7. Composition cosmétique selon l'une quelconque des revendications précédentes, **caractérisée en ce que** le ou les tensioactifs anioniques sont choisis parmi les alkylsulfates, les alkyléthersulfates et les alkyléthercarboxylates, et leurs mélanges, en particulier sous forme de sels de métaux alcalins ou alcalino-terreux, d'ammonium, d'amine ou d'aminoalcool.

8. Composition cosmétique selon l'une quelconque des revendications précédentes, **caractérisée en ce que** la ou les silicones aminées est ou sont choisies parmi :
(a) les composés répondant à la formule (IV) suivante :
(R¹)ₐ(T)₃₋ₐ-Si[OSi(T)₂]ₙ-[OSi(T)_{b}(R¹)_{2-b}]ₘ-OSi(T)₃₋ₐ-(R¹)ₐ (IV)
dans laquelle,
T est un atome d'hydrogène, ou un radical phényle, hydroxyle (-OH), ou alkyle en C₁-C₈, et de préférence méthyle ou alcoxy en C₁-C₈, de préférence méthoxy,
a désigne le nombre 0 ou un nombre entier de 1 à 3, et de préférence 0,
b désigne 0 ou 1, et en particulier 1,
m et n sont des nombres tels que la somme (n + m) peut varier notamment de 1 à 2 000 et en particulier de 50 à 150, n pouvant désigner un nombre de 0 à 1 999 et notamment de 49 à 149 et m pouvant désigner un nombre de 1 à 2 000, et notamment de 1 à 10 ;
R₁ est un radical monovalent de formule -C_{q}H_{2q}L dans laquelle q est un nombre de 2 à 8 et L est un groupement aminé choisi parmi les groupements :
-N(R²)-CH₂-CH₂-N(R²)₂ ;
-N(R²)₂ ;
dans lesquels R² peut désigner un atome d'hydrogène, un radical phényle, un radical benzyle, ou un radical hydrocarboné saturé monovalent, par exemple un radical alkyle en C₁-C₂₀ et
b) les silicones aminées de formule (VII) : 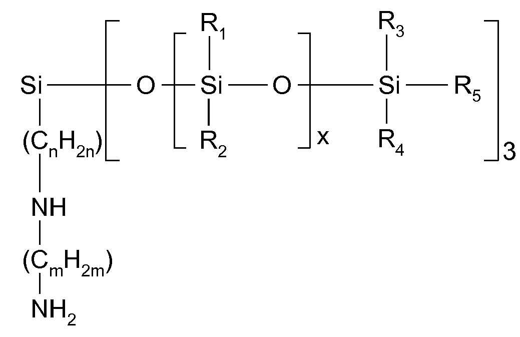 dans laquelle :
- R₁, R₂, R₃ et R₄, identiques ou différents, désignent un radical alkyle en C₁-C₄ ou un groupement phényle,
- R₅ désigne un radical alkyle en C₁-C₄ ou un groupement hydroxyle,
- n est un entier variant de 1 à 5,
- m est un entier variant de 1 à 5,
et dans laquelle x est choisi de manière telle que l'indice d'amine soit compris entre 0,01 et 1 meq/g
c) le POLYSILICON-9.

9. Composition cosmétique selon la revendication 8, **caractérisée en ce que** la silicone aminée correspondant à la formule (IV) est choisie parmi les composés correspondant à la formule suivante : 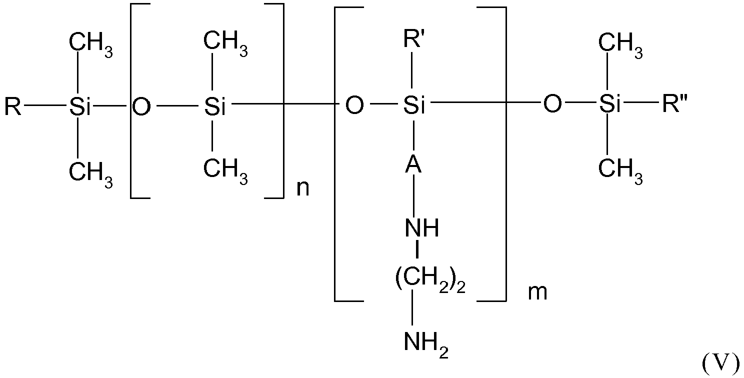 dans laquelle R, R', R", identiques ou différents, désignent un radical alkyle en C₁-C₄, de préférence CH₃ ; un radical alcoxy en C₁-C₄, de préférence méthoxy ; ou OH ; A représente un radical alkylène, linéaire ou ramifié, en C₃-C₈, de préférence en C₃-C₆; m et n sont des nombres entiers dépendant du poids moléculaire et dont la somme est comprise entre 1 et 2000.

10. Composition cosmétique selon la revendication 8, **caractérisée en ce que** la silicone aminée de formule (IV) est la silicone dénommée "triméthylsilylamodiméthicone", répondant à la formule (VI) suivante: 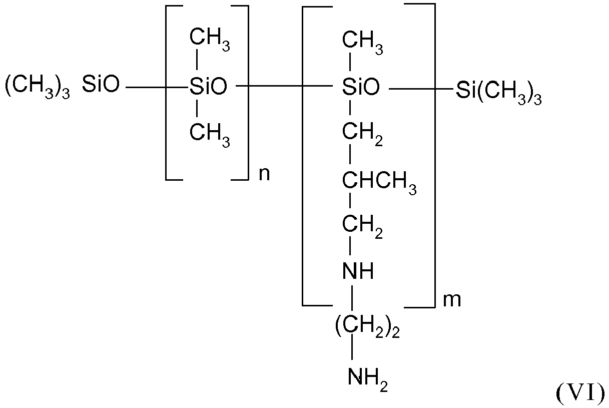 dans laquelle n et m ont les significations données ci-dessus conformément à la formule (IV).

11. Composition cosmétique selon l'une quelconque des revendications précédentes, **caractérisée en ce qu'**elle comprend en outre un ou plusieurs acides organiques.

12. Composition cosmétique selon l'une quelconque des revendications précédentes, **caractérisée en ce qu'**elle comprend en outre un ou plusieurs tensioactifs additionnels choisis parmi les tensioactifs amphotères et les tensioactifs non ioniques.

13. Composition cosmétique selon l'une quelconque des revendications précédentes, **caractérisée en ce qu'**elle comprend en outre un ou plusieurs polymères cationiques.

14. Procédé de traitement cosmétique des fibres kératiniques, en particulier des fibres kératiniques humaines telles que les cheveux, **caractérisé en ce que** l'on applique la composition cosmétique telle que définie selon l'une quelconque des revendications précédentes, sur lesdites fibres, et **en ce que** l'on rince après un éventuel temps de pose.

15. Utilisation d'une composition selon l'une quelconque des revendications 1 à 13, comme shampooing, pour le nettoyage et le conditionnement des fibres kératiniques.

## Patentansprüche

1. Kosmetische Zusammensetzung zur Reinigung und Konditionung von Keratinfasern, insbesondere menschlichen Keratinfasern wie Haaren, die in einem kosmetisch akzeptablen Medium Folgendes umfasst:
i) eine oder mehrere organische Siliciumverbindungen, ausgewählt aus Silanen, die ein Siliciumatom umfassen, und Siloxanen, die zwei oder drei Siliciumatome umfassen, wobei die organischen Siliciumverbindungen ferner eine oder mehrere chemische basische funktionelle Gruppen und eine oder mehrere Hydroxylgruppen oder hydrolysierbare Gruppen pro Molekül aufweisen;
ii) ein oder mehrere anionische Tenside; und
iii) ein oder mehrere nicht quaternisierte Aminosilikone, die von den Verbindungen i) verschieden sind.

2. Kosmetische Zusammensetzung nach Anspruch 1, **dadurch gekennzeichnet, dass** die chemischen basischen funktionellen Gruppen der organischen Siliciumverbindung aus primären, sekundären oder tertiären Aminen ausgewählt sind.

3. Kosmetische Zusammensetzung nach Anspruch 1 oder 2, **dadurch gekennzeichnet, dass** die hydrolysierbaren Gruppen aus Alkoxy-, Aryloxy- und Halogengruppen ausgewählt sind.

4. Kosmetische Zusammensetzung nach einem der Ansprüche 1 bis 3, **dadurch gekennzeichnet, dass** die organische(n) Siliciumverbindung(en) ausgewählt sind aus Verbindungen mit der Formel (I): 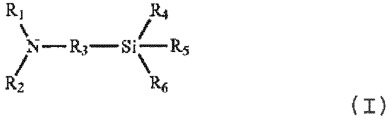 wobei:
R₄ ein Halogen, eine Gruppe OR' oder R'₁ bedeutet;
R₅ ein Halogen, eine Gruppe OR" oder R'₂ bedeutet;
R₆ ein Halogen, eine Gruppe OR"' oder R'₃ bedeutet;
R₁, R₂, R₃, R', R", R"', R'₁, R'₂, R'₃ unabhängig voneinander eine lineare oder verzweigte, gesättigte oder ungesättigte Kohlenwasserstoffgruppe bedeuten, die gegebenenfalls zusätzliche chemische Gruppen trägt,
wobei R₁, R₂, R', R" und R"' ferner Wasserstoff bezeichnen können, und mindestens zwei der Gruppen R₄,
R₅ und R₆ jeweils OR', OR" und OR"' bezeichnen, wobei mindestens zwei der Gruppen R', R" und R"' von
Wasserstoff verschieden sind; und 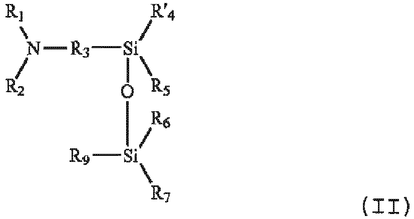 wobei:
R₁, R₂, R₃, R₅ und R₆ wie oben definiert sind;
R'₄ ein Halogenatom oder eine Gruppe OR₁₁ bedeutet;
R₇ ein Halogenatom oder eine Gruppe OR₁₀ oder R"₁ bedeutet;
R₉ ein Halogenatom, eine Gruppe OR₈, R"₂ oder R₃NR₁R₂ bedeutet;
R"₁, R"₂, R₈, R₁₀ und R₁₁ eine lineare oder verzweigte, gesättigte oder ungesättigte Kohlenwasserstoffgruppe bedeuten, die gegebenenfalls zusätzliche chemische Gruppen trägt, wobei die Gruppen R₁₁, R₁₀ und R₈ ferner ein Wasserstoffatom bedeuten können; wobei mindestens eine der Gruppen R₆, R₇ und R₉ ein Wasserstoffatom, eine Gruppe OR"', OR₁₀ oder OR₈ bezeichnet.

5. Kosmetische Zusammensetzung nach Anspruch 4, **dadurch gekennzeichnet, dass** die Gruppen R₁, R₂, R', R'₁, R'₂, R'₃, R", R"', R"₁, R"₂, R₈, R₁₀ und R₁₁ aus C₁-bis C₁₂-Alkylresten, C₆- bis C₁₄-Arylresten, C₁-C₈-Alkyl-C₆-C₁₄-Arylresten und C₆-C₁₄-Aryl-C₁-C₈-Alkylresten ausgewählt sind.

6. Kosmetische Zusammensetzung nach einem der vorhergehenden Ansprüche, **dadurch gekennzeichnet, dass** die organische(n) Siliciumverbindung(en) ausgewählt sind aus Verbindungen mit der Formel (III): 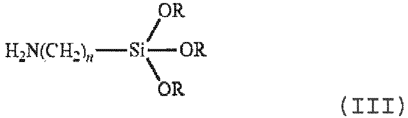 wobei die Reste R, gleich oder verschieden, aus C₁- bis C₆-Alkylresten ausgewählt sind, und n eine ganze Zahl von 1 bis 6, vorzugsweise von 2 bis 4 ist.

7. Kosmetische Zusammensetzung nach einem der vorhergehenden Ansprüche, **dadurch gekennzeichnet, dass** das oder die anionische(n) Tensid(e) ausgewählt sind aus Alkylsulfaten, Alkylethersulfaten und Alkylethercarboxylaten sowie ihren Mischungen, insbesondere in Form von Alkali- oder Erdalkalimetallsalzen, Ammonium-, Amin- oder Aminoalkohol-Salzen.

8. Kosmetische Zusammensetzung nach einem der vorhergehenden Ansprüche, **dadurch gekennzeichnet, dass** das oder die Aminosilikon(e)ausgewählt ist bzw. sind aus Folgendem:
(a) Verbindungen, die folgender Formel (IV) entsprechen:
(R¹)ₐ (T) ₃₋ₐ-Si [OSi (T)₂]ₙ-[OSi(T) _{b}(R¹)_{2-b}]ₘ-OSi(T) ₃₋ₐ-(R¹)ₐ (IV)
wobei
T ein Wasserstoffatom oder ein Phenyl-, Hydroxyl- (-OH) oder C₁- bis C₈-Alkylrest, und vorzugsweise Methyl, oder
C₁- bis C₈-Alkoxyrest, vorzugsweise Methoxy, ist,
a die Zahl 0 oder eine ganze Zahl von 1 bis 3 und
vorzugsweise 0 bezeichnet,
b 0 oder 1 und insbesondere 1 bezeichnet,
m und n derartige Zahlen sind, dass die Summe (n + m) insbesondere von 1 bis 2000 und insbesondere von 50 bis 150 variieren kann, n eine Zahl von 0 bis 1999 und insbesondere von 49 bis 149 bezeichnen kann, und m eine Zahl von 1 bis 2000 und insbesondere von 1 bis 10 bezeichnen kann;
R₁ ein einwertiger Rest mit der Formel -C_{q}H_{2q}L ist, wobei q eine Zahl von 2 bis 8 ist und L eine Aminogruppe ist,
die aus folgenden Gruppen ausgewählt ist:
- N(R²)CH₂-CH₂-N(R²)₂ ;
- N(R²)₂;
wobei R² ein Wasserstoffatom, einen Phenylrest, einen Benzylrest oder einen gesättigten einwertigen Kohlenwasserstoffrest bezeichnen kann, beispielsweise einen C₁- bis C₂₀-Alkylrest, und
b) Aminosilikonen mit der Formel (VII): 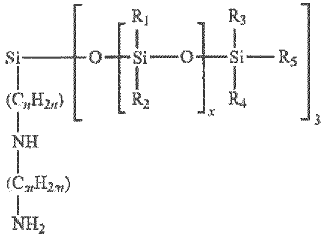 wobei:
- R₁, R₂, R₃ und R₄, gleich oder verschieden, einen C₁-bis C₄-Alkylrest oder eine Phenylgruppe bezeichnen,
- R₅ einen C₁- bis C₄-Alkylrest oder eine Hydroxylgruppe bezeichnet,
- n eine ganze Zahl von 1 bis 5 ist,
- m eine ganze Zahl von 1 bis 5 ist,
und wobei x so ausgewählt ist, dass die Aminzahl zwischen 0,01 und 1 meq/g liegt,
c) POLYSILICON-9.

9. Kosmetische Zusammensetzung nach Anspruch 8, **dadurch gekennzeichnet, dass** das Aminosilikon, das der Formel (IV) entspricht, aus den Verbindungen ausgewählt ist, die folgender Formel entsprechen: 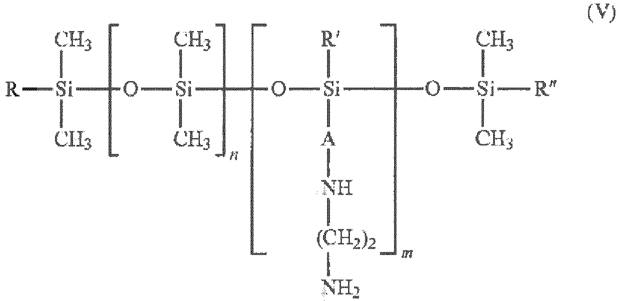 wobei R, R', R", gleich oder verschieden, einen C₁- bis C₄-Alkylrest, vorzugsweise CH₃; einen C₁- bis C₄-Alkoxyrest, vorzugsweise Methoxy; oder OH bezeichnen; A einen linearen oder verzweigten C₃- bis C₈-Alkylenrest, vorzugsweise C₃- bis C₆-Alkylenrest, bedeutet; m und n von der Molekülmasse abhängige ganze Zahlen sind, deren Summe zwischen 1 und 2000 liegt.

10. Kosmetische Zusammensetzung nach Anspruch 8, **dadurch gekennzeichnet, dass** das Aminosilikon mit der Formel (IV) das als "Trimethylsilylamodimeticon" bezeichnete Silikon ist, das folgender Formel (VI) entspricht: 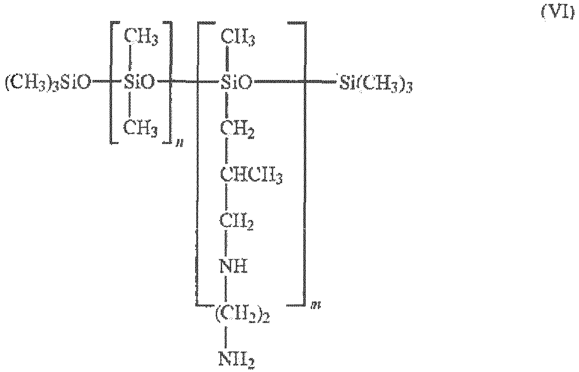 wobei n und m die oben angegebene Bedeutung entsprechend Formel (IV) aufweisen.

11. Kosmetische Zusammensetzung nach einem der vorhergehenden Ansprüche, **dadurch gekennzeichnet, dass** sie ferner eine oder mehrere organische Säuren umfasst.

12. Kosmetische Zusammensetzung nach einem der vorhergehenden Ansprüche, **dadurch gekennzeichnet, dass** sie ferner ein oder mehrere zusätzliche Tenside umfasst, die aus amphoteren Tensiden und nichtionischen Tensiden ausgewählt sind.

13. Kosmetische Zusammensetzung nach einem der vorhergehenden Ansprüche, **dadurch gekennzeichnet, dass** sie ferner ein oder mehrere kationische Polymere umfasst.

14. Verfahren zum kosmetischen Behandeln von Keratinfasern, insbesondere menschlichen Keratinfasern wie Haaren, **dadurch gekennzeichnet, dass** die kosmetische Zusammensetzung, wie sie nach einem der vorhergehenden Ansprüche definiert ist, auf die Fasern aufgetragen wird, und dass sie nach einer etwaigen Einwirkzeit ausgespült wird.

15. Verwendung einer Zusammensetzung nach einem der Ansprüche 1 bis 13 als Shampoo, zum Reinigen und Konditionen von Keratinfasern.

## Claims

1. Cosmetic composition for cleaning and conditioning keratin fibres, in particular human keratin fibres such as the hair, comprising in a cosmetically acceptable medium:
i) one or more organic silicon compounds selected from silanes comprising one silicon atom and siloxanes comprising two or three silicon atoms, said organic silicon compounds moreover having one or more basic chemical functions and one or more hydroxyl groups or hydrolysable groups per molecule;
ii) one or more anionic surfactants; and
iii) one or more non-quaternized aminated silicones different from compounds i).

2. Cosmetic composition according to Claim 1, **characterized in that** the basic chemical functions of the organic silicon compound are selected from primary, secondary or tertiary amines.

3. Cosmetic composition according to Claim 1 or 2, **characterized in that** the hydrolysable groups are selected from alkoxy, aryloxy and halogen groups.

4. Cosmetic composition according to any one of Claims 1 to 3, **characterized in that** the organic silicon compound or compounds are selected from the compounds of formula (I): 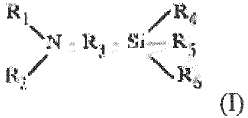 in which:
R₄ represents a halogen, a group OR' or R'₁;
R₅ represents a halogen, a group OR" or R'₂;
R₆ represents a halogen, a group OR"' or R'₃;
R₁, R₂, R₃, R', R", R'", R'₁, R'₂, R'₃ represent, independently of one another, a saturated or unsaturated, linear or branched hydrocarbon group, optionally bearing additional chemical groups, where R₁, R₂, R', R" and R"' can moreover denote hydrogen, and at least two of the groups R₄, R₅ and R₆ denote respectively OR', OR" and OR"', at least two of the groups R', R" and R'" being different from hydrogen; and 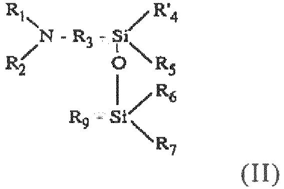 in which:
R₁, R₂, R₃, R₅ and R₆ are defined as previously;
R'₄ represents a halogen atom or a group OR₁₁;
R₇ represents a halogen atom, a group OR₁₀ or R"₁;
R₉ represents a halogen atom, a group OR₈, R"₂ or R₃NR₁R₂;
R"₁, R"₂, R₈, R₁₀ and R₁₁ represent a saturated or unsaturated, linear or branched hydrocarbon group, optionally bearing additional chemical groups, and groups R₁₁, R₁₀ and R₈ can moreover represent a hydrogen atom; at least one of groups R₆, R₇ and R₉ denoting a halogen atom, a group OR"', OR₁₀ or OR₈.

5. Cosmetic composition according to Claim 4, **characterized in that** groups R₁, R₂, R', R'₁, R'₂, R'₃, R", R"', R"₁, R"₂, R₈, R₁₀ and R₁₁ are selected from C₁-C₁₂ alkyl, C₆ to C₁₄ aryl, C₁ to C₈ alkyl-C₆ to C₁₄ aryl, and C₆ to C₁₄ aryl-C, to C₈ alkyl radicals.

6. Cosmetic composition according to any one of the preceding claims, **characterized in that** the organic silicon compound or compounds are selected from the compounds of formula (III): 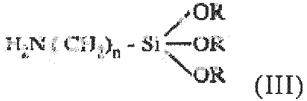 in which the radicals R, which may be identical or different, are selected from C₁-C₆ alkyl radicals and n is an integer from 1 to 6, preferably from 2 to 4.

7. Cosmetic composition according to any one of the preceding claims, **characterized in that** the anionic surfactant or surfactants are selected from alkylsulphates, alkylethersulphates and alkylethercarboxylates, and mixtures thereof, in particular in the form of salts of alkali metals or alkaline-earth metals, of ammonium, of amine or of amino alcohol.

8. Cosmetic composition according to any one of the preceding claims, **characterized in that** the aminated silicone or silicones is/are selected from:
(a) the compounds corresponding to the following formula (IV):
(R¹)ₐ(T)₃₋ₐ-Si[OSi(T)₂]ₙ-[OSi(T)_{b}(R¹)_{2-b}]ₘ-OSi(T)₃₋ₐ-(R¹)ₐ (IV)
in which,
T is a hydrogen atom, or a phenyl, hydroxyl (-OH), or C₁-C₈ alkyl radical, and preferably methyl or C₁-C₈ alkoxy, preferably methoxy,
a denotes the number 0 or an integer from 1 to 3, and preferably 0,
b denotes 0 or 1, and in particular 1,
m and n are numbers such that the sum (n + m) can vary notably from 1 to 2000 and in particular from 50 to 150, and n can denote a number from 0 to 1999 and notably from 49 to 149 and m can denote a number from 1 to 2000, and notably from 1 to 10;
R₁ is a monovalent, radical of formula -C_{q}H_{2q}L in which q is a number from 2 to 8 and L is an amino group selected from the groups:
-N(R²)-CH₂-CH₂-N(R²)₂;
-N(R²)₂;
in which R² can denote a hydrogen atom, a phenyl radical, a benzyl radical, or a saturated monovalent hydrocarbon radical, for example a C₁-C₂₀ alkyl radical and
b) the aminated silicones of formula (VII): 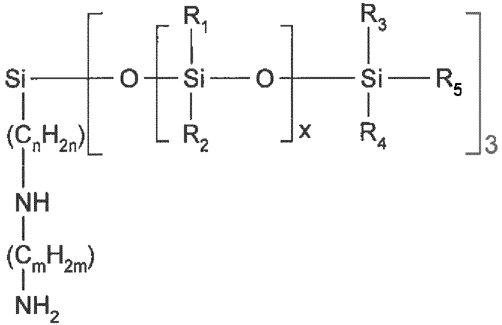 in which:
- R₁, R₂, R₃ and R₄, which may be identical or different, denote a C₁-C₄ alkyl radical or a phenyl group,
- R₅ denotes a C₁-C₄ alkyl radical or a hydroxyl group,
- n is an integer in the range from 1 to 5,
- m is an integer in the range from 1 to 5,
and in which x is selected in such a way that the amine index is between 0.01 and 1 meq/g
c) POLYSILICON-9.

9. Cosmetic composition according to Claim 8, **characterized in that** the aminated silicone corresponding to formula (IV) is selected from the compounds corresponding to the following formula: 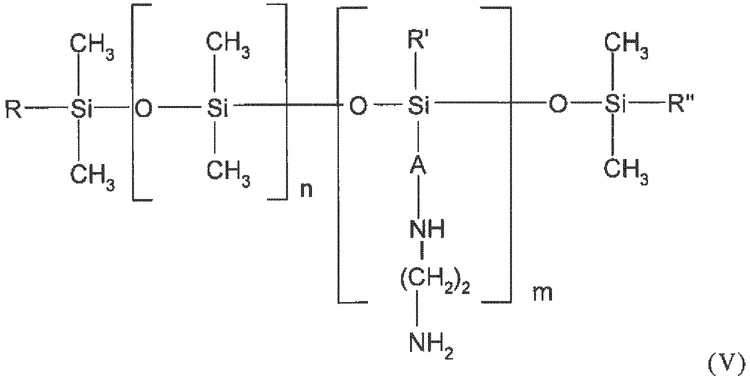 in which R, R', R", which may be identical or different, denote a C₁-C₄ alkyl radical, preferably CH₃; a C₁-C₄ alkoxy radical, preferably methoxy; or OH; A represents a linear or branched, C₃-C₈, preferably C₃-C₆ alkylene radical; m and n are integers depending on the molecular weight and whose sum is between 1 and 2000.

10. Cosmetic composition according to Claim 8, **characterized in that** the aminated silicone of formula (IV) is the silicone called "trimethylsilylamodimethicone", corresponding to the following formula (VI): 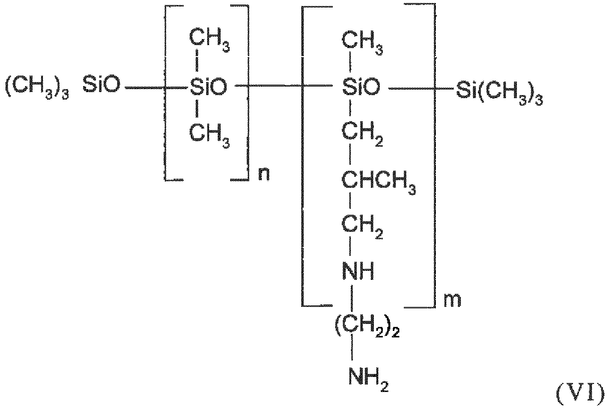 in which n and m have the meanings given above according to formula (IV).

11. Cosmetic composition according to any one of the preceding claims, **characterized in that** it further comprises one or more organic acids.

12. Cosmetic composition according to any one of the preceding claims, **characterized in that** it further comprises one or more additional surfactants selected from amphoteric surfactants and non-ionic surfactants.

13. Cosmetic composition according to any one of the preceding claims, **characterized in that** it further comprises one or more cationic polymers.

14. Method of cosmetic treatment of keratin fibres, in particular of human keratin fibres such as the hair, **characterized in that** the cosmetic composition as defined according to any one of the preceding claims is applied on said fibres, followed by rinsing after an optional pause.

15. Use of a composition according to any one of Claims 1 to 13, as shampoo, for cleaning and conditioning keratin fibres.
